# EUROPEAN PATENT APPLICATION

(11) **EP 4 324 464 A1**
(43) Date of publication of application: **21.02.2024**
(21) Application number: 22787365.0
(22) Date of filing: 25.03.2022
(51) Int. Cl.: A61K 31/52, A61P 17/02, A61P 3/10

(54) **USE OF ADENINE IN PREPARATION OF DRUG FOR TREATING DIABETIC ULCERS**

(30) Priority: 16.04.2021 CN 202110411356
(71) Applicant: Energenesis Biomedical Co., Ltd., 11492 Taipei (TW)
(72) Inventor: CHIO, Jen-yi, TAIPEI, Taiwan 11492 (TW); CHEN, Han-min, TAIPEI, Taiwan 11492 (TW); LIN, Jiun-tsai, TAIPEI, Taiwan 11492 (TW); CHENG, Yi-fang, TAIPEI, Taiwan 11492 (TW); YOUNG, Guang-huar, TAIPEI, Taiwan 11492 (TW); HUANG, Chun-fang, TAIPEI, Taiwan 11492 (TW)
(74) Representative: Epping - Hermann - Fischer
(86) International application number: PCT/CN2022/083129
(87) International publication number: WO 2022/218136

(57) **Abstract**

The present disclosure provides a use of a pharmaceutical composition including adenine and/or a pharmaceutically acceptable salt thereof in a manufacture of a medicament for treating diabetic ulcers, and the medicament can effectively accelerate and enhance wound healing of diabetic ulcers and prevent scar formation.

## Description

### BACKGROUND

### 1. Technical Field

The present disclosure relates to a use of adenine in the preparation of medicaments for treating diabetic ulcers, particularly for treating diabetic foot ulcers.

### 2. Description of the Related Art

Wound healing, a dynamic tissue remodeling process, involves formation of a matrix rich in fibrin and fibronectin, infiltration of neutrophils and macrophages, proliferation of epithelial keratinocytes at the wound edges and their migration through a provisional matrix, the formation of granulation tissue containing migrating inflammatory cells and fibroblasts, wound contraction, etc. Ideally, wound healing refers to the wound with restoration of normal anatomy, function, and appearance at the levels of cells, tissue, organs, and organisms. If appropriate treatment is performed, wound healing usually takes one to two weeks.

Currently, delayed healing of diabetic ulcers remains unmet needs for wound care and tissue repair. Diabetic ulcers, such as diabetic foot ulcers, cannot be healed in a normal or expectative speed are a major complication of diabetes, and can lead to lower limb amputation. Hence, diabetic ulcers are a growing global problem that seriously affects the life quality of many individuals and may even result in death.

Furthermore, diabetic ulcers can be distinguished into different severity depending on the condition of the wound of diabetic ulcer, such as depth of the wound, area of necrotic wound or tissue, degree of ischemia of wound, or degree of infection of wound. Different grades of diabetic ulcers may require different treatment approaches, and therefore, there is still a need for drugs and methods effective for treating diabetic ulcers of different grades.

Hence, there is an urgent need in the art for medications and their uses thereof that can address the above problems.

### SUMMARY

The present disclosure provides a use of a pharmaceutical composition comprising adenine and/or a pharmaceutically acceptable salt thereof in the manufacture of a medicament for treating diabetic ulcers.

The present disclosure further provides a pharmaceutical composition comprising adenine and/or a pharmaceutically acceptable salt thereof for use in the treatment of diabetic ulcers.

According to at least one embodiment of the present disclosure, the diabetic ulcer is one of Grades 1 to 3 in Wagner-Meggitt ulcer classification, such as Grade 1, Grade 2, or Grade 3.

According to at least one embodiment of the present disclosure, the diabetic ulcer is Grade 1 or Grade 2 in the Wagner-Meggitt ulcer classification system.

According to at least one embodiment of the present disclosure, the diabetic ulcer is located on a skin, a mouth, a gums, a corneal epithelial tissue, or any combination of two or more thereof.

According to at least one embodiment of the present disclosure, an area of the diabetic ulcer is in a range from 1.5 to 25 square centimeters.

According to at least one embodiment of the present disclosure, the diabetic ulcer is a wound without ischemic and infection, a wound with an ischemic and without infection, a wound without ischemic and with infection, or a wound with ischemic and infection.

According to at least one embodiment of the present disclosure, the adenine and/or a pharmaceutically acceptable salt thereof is a single active ingredient in the pharmaceutical composition.

According to at least one embodiment of the present disclosure, the adenine and/or a pharmaceutically acceptable salt thereof in the pharmaceutical composition is in an amount of 0.0001 wt% to 2 wt%.

According to at least one embodiment of the present disclosure, the pharmaceutical composition is formulated to a form selected from a group consisting of solutions, ointments, detergents, sprays, aerosols, creams, foams, lotions, gels, pastes, patches, adhesives, glues, films, intraoral pastes, powders, and wound dressings.

According to at least one embodiment of the present disclosure, the pharmaceutical composition further comprises a pharmaceutically acceptable carrier or excipient.

According to at least one embodiment of the present disclosure, an effective dosage of the adenine and/or a pharmaceutically acceptable salt thereof is in a range from 0.1 mg/kg body weight to 50 mg/kg body weight.

According to at least one embodiment of the present disclosure, the adenine and/or a pharmaceutically acceptable salt thereof is administered to a subject through a topical administration or a systemic administration.

In addition to wound healing, reducing scar formation during wound healing is always a primary goal in modern medical science. Studies have found that unlike wound healing in adults, wound healing in newborns would not easily form scars, and the difference lies in the activation of cyclooxygenase-2 (COX-2). COX-2 activity is enhanced by TGF-β during wound healing in adults, and production of at the wound area is increased. Prostaglandins is identified as promoting fibroblast proliferation and collagen formation, both of which contribute to scar formation. Therefore, inhibiting the activity of COX-2 is considered to effectively prevent scar formation. The inventors conducted extensive research and found that adenine can inhibit fibroblast growth and reduce protein expression of COX-2. After directly administering adenine to ulcer wounds in diabetic patients, not only wound healing is promoted, but also scar formation is reduced. Based on the above information, topical administration of adenine is effective in accelerating and enhancing wound healing of diabetic ulcers and preventing scar formation.

According to at least one embodiment of the present disclosure, the adenine and/or a pharmaceutically acceptable salt thereof can be used for anti-inflammatory purposes, increasing collagen secretion, promoting epithelial cell migration, inhibiting scar formation, inhibiting fibroblast growth, or any combination of two or more of these effects.

### DETAILED DESCRIPTION OF THE PREFERRED EMBODIMENTS

The following examples are provided for illustrative purposes to describe the content of this disclosure. Those skilled in the art of the relevant technical field will understand other advantages of this disclosure based on the description provided in the specification. This disclosure can be implemented or applied as described in other embodiments. Modifications and changes can be made to such embodiments for different embodiments and applications within the scope of this disclosure.

It should be noted that the singular forms "a," "an," and "the" used herein include the plural forms unless specifically limited to the singular form. The term "or" and the term "and/or" are used interchangeably unless otherwise indicated.

The numerical ranges described herein are inclusive and combinable. Any value within the numerical ranges described herein can be used as the maximum or minimum value to define a subrange. For example, the numerical range "0.0001 wt% to 2 wt%" should be understood to include any subrange between the minimum value of 0.0001 wt% and the maximum value of 2 wt%, such as 0.0001 wt% to 1.5 wt%, 0.01 wt% to 2 wt%, or 0.005 wt% to 1 wt%. Additionally, multiple numerical endpoints described herein can be selected as the maximum or minimum value to define a numerical range. For example, 0.0001, 0.01, or 50 mg/kg body weight can define a numerical range of 0.0001 to 0.01 mg/kg body weight, 0.0001 to 50 mg/kg body weight, or 0.01 to 50 mg/kg body weight.

The term "diabetes" used herein refers to type 1 diabetes, type 2 diabetes, gestational diabetes, cystic fibrosis-related diabetes (CFRD), latent autoimmune diabetes in adults (LADA), monogenic diabetes, or any other form of diabetes, but is not limited thereto.

The term "ulcer" used herein refers to an open pathological condition characterized by non-healing or slow-healing internal or external surface lesions (e.g., wounds) caused by mucosal or skin damage or rupture. Ulcers can occur on the body's internal or external surfaces, including but not limited to oral cavity, gastrointestinal tract, cornea, or skin. Common types of ulcers include pit-like lesions caused by inflammatory, infectious, malignant, or metabolic disorders. The term "diabetic ulcer" used herein refers to ulcers resulting from complications of diabetes that typically cannot heal within 1 to 3 months. Diabetic ulcers are commonly found on the extremities, particularly the legs or feet. The term "diabetic foot ulcer" used herein refers to ulcers formed on the patient's leg or foot, including but not limited to ulcers on the thigh, groin, knee, calf, shin, ankle, heel, sole, toes, surrounding areas thereof, or any combination of two or more thereof.

In at least one embodiment of the present disclosure, diabetic patients with exemplary diabetic ulcers are susceptible to ulceration due to neurological and vascular complications. For example, peripheral neuropathy leads to sensory alterations or complete loss in the foot and/or leg, which may result in the inability to perceive minor foot cuts or injuries, lack of awareness of sustained pressure damage, etc., ultimately leading to tissue ischemia and necrosis, thereby causing ulcers, such as plantar ulcers. Additionally, microvascular diseases are also significant complications in diabetic patients and may contribute to ulcer.

To address the problems in the prior art, the present disclosure provides a use of adenine and/or a pharmaceutically acceptable salt thereof in preparation of a medicament for treating diabetic ulcers.

Currently, there are several widely used systems for the systematic classification of diabetic ulcers, with the Wagner-Meggitt ulcer classification system being commonly employed, which classifies ulcers based on the depth of the wound and the extent of tissue necrosis. Specifically, superficial ulcers are categorized as Grade 1; deep ulcers (e.g., involving ligaments, tendons, bones, or joints) are classified as Grade 2; deep ulcers with the presence of abscess or osteomyelitis are designated as Grade 3; ulcers accompanied by forefoot gangrene are categorized as Grade 4; and ulcers with complete foot gangrene are classified as Grade 5. In the present disclosure, when adenine and/or a pharmaceutically acceptable salt thereof is applied to diabetic ulcers of Grade 1 or Grade 2 according to the Wagner-Meggitt ulcer classification system, a significant therapeutic effect is observed, the period of wound healing is reduced, and the ratio of complete healing is increased.

In at least one embodiment, the subject can be mammals, such as human or mice. In at least one embodiment, the mammal is human. In other embodiments, the mammal can be a non-human mammal.

In at least one embodiment, the adenine and/or a pharmaceutically acceptable salt thereof shows a significant therapeutic effect when applied to diabetic ulcer wounds with an area ranging from 1.5 to 25 square centimeters. The wound area can be about 1.5, about 2, about 2.5, about 3, about 3.5, about 4, about 4.5, about 5, about 5.5, about 6, about 6.5, about 7, about 7.5, about 8, about 8.5, about 9, about 9.5, about 10, about 11, about 12, about 13, about 14, about 15, about 16, about 17, about 18, about 19, about 20, about 21, about 22, about 23, about 24, about 25, about 30, about 40, or about 50 square centimeters, but is not limited thereto. Moreover, multiple numerical endpoints mentioned above can be selectively chosen as maximum or minimum values to define the range. In at least one embodiment, adenine and/or a pharmaceutically acceptable salt thereof demonstrates a significant therapeutic effect when applied to diabetic ulcer wounds with an area ranging from 5 to 25 square centimeters, 10 to 25 square centimeters, 15 to 25 square centimeters, or 1.5 to 20 square centimeters.

In at least one embodiment, adenine and/or a pharmaceutically acceptable salt thereof can be administered to diabetic ulcers located on the skin, oral cavity, gums, corneal epithelium, or any combination thereof.

On the other hand, in addition to the Wagner-Meggitt ulcer classification system mentioned above for evaluating diabetic ulcers, the University of Texas System is also commonly used as a classification system for ulcers. The University of Texas System evaluates both the depth of the wound and the presence of ischemia and infection. Specifically, Stage A indicates a non-ischemic and non-infected wound (clean wounds); Stage B indicates a non-ischemic infected wounds; Stage C indicates an ischemic non-infected wound; Stage D indicates an ischemic infected wound; Grade 1 indicates a superficial wound; Grade 2 indicates a shallow wound; Grade 3 indicates a deep wound involving tendons or synovial cavities; Grade 4 indicates a deep wound involving bone or joint. In at least one embodiment of the present disclosure, the diabetic ulcer is a non-ischemic and non-infected wound, an ischemic non-infected wound, a non-ischemic infected wound, or an ischemic infected wound.

In at least one embodiment, the ankle brachial pressure index (ABPI) of the subject can range from about 0.1, about 0.2, about 0.3, about 0.4, about 0.5, about 0.6, about 0.7, about 0.8, about 0.9, about 1.0, about 1.1, about 1.2, about 1.3, about 1.4, about 1.0, about 1.5, about 1.6, about 1.7, about 1.8, about 1.9, but is not limited thereto. Additionally, various numerical endpoints mentioned above may be selected as maximum or minimum values to define the range of values. In at least one embodiment, the ankle brachial pressure index of the subject is from 0.4 to 1.8 or from 0.4 to 1.5. In at least one embodiment, the subject with ischemic wounds has an ankle brachial pressure index less than 1 or less than 0.4.

In at least one embodiment disclosed herein, when adenine and/or a pharmaceutically acceptable salt thereof are applied to the wounds of diabetic ulcers, the adenine and/or a pharmaceutically acceptable salt thereof can also be used to reduce inflammation, increase collagen secretion, promote migration of epithelial cells, inhibit scar formation, inhibit fibroblast growth, promote matrix deposition, or any combination thereof, thereby enhancing epithelial regeneration of the wound, and effectively improving and enhancing the healing of diabetic ulcers. In some embodiments, the adenine and/or a pharmaceutically acceptable salt thereof can be used to inhibit fibroblast proliferation, thereby preventing scar formation during the healing of diabetic ulcers.

In at least one embodiment disclosed herein, adenine and/or a pharmaceutically acceptable salt thereof is used in the form of a composition for treating wounds of diabetic ulcers, and in some embodiments, the adenine and/or a pharmaceutically acceptable salt thereof serve as a single active ingredient in the composition. The composition can be administered once a day for a period of 7 to 30 days during treatment. In some embodiments, the composition can be administered once a day for a period of 14 to 28 days. In other embodiments, the composition can be administered one to four times a day for a period of 1 to 4 months, for example, twice a day for a period of 3 to 4 months.

In at least one embodiment disclosed herein, the content of adenine and/or a pharmaceutically acceptable salt thereof, serving as an active ingredient in the composition, ranges from 0.0001 wt% to 2 wt%. In at least one embodiment, the amount of adenine and/or a pharmaceutically acceptable salt thereof in the composition can range from 0.0001 wt% to 1 wt%. In some embodiments, the amount of adenine and/or a pharmaceutically acceptable salt thereof in the composition can range from 0.0001 wt% to 0.05 wt%. In some embodiments, the amount of adenine and/or a pharmaceutically acceptable salt thereof in the composition can range from 0.001 wt% to 1 wt%. In some embodiments, the amount of adenine and/or a pharmaceutically acceptable salt thereof in the composition can range from 0.005 wt% to 0.08 wt%. In other embodiments, the amount of adenine and/or a pharmaceutically acceptable salt thereof in the composition can range from 0.005 wt% to 0.02 wt%. In some embodiments, the amount of adenine and/or a pharmaceutically acceptable salt thereof in the composition can be about 0.0001%, about 0.001%, about 0.005%, about 0.01%, about 0.05%, about 0.1%, about 0.2%, about 0.3%, about 0.4%, about 0.5%, about 0.6%, about 0.7%, about 0.8%, about 0.9%, about 1%, about 1.2%, about 1.5%, about 1.8%, or about 2 wt%. Additionally, multiple numerical endpoints mentioned above can be selected as maximum or minimum values to define the range of values.

In at least one embodiment disclosed herein, the composition can be formulated in a form selected from a group consisting of solution, ointment, lotion, spray, aerosol, cream, foam, gel, paste, patch, adhesive, adhesive gel, film, oral paste, powder, and wound dressing.

In at least one embodiment disclosed herein, the composition may include, but is not limited to, other components acceptable in pharmaceuticals, such as carriers, excipients, adjuvants, and/or formulation agents. For example, the composition may include, but is not limited thereto, any combination of one or more than two selected from a group consisting of gel-forming agents, solvents, cosolvents, stabilizers, tensionenhancing agents, permeation enhancers, pH-adjusting agents, humectants, surfactants, buffers, preservatives, viscosity modifiers, emulsifiers, propellants, suspending agents, flavoring agents, coloring agents, and antimicrobial agents.

In at least one embodiment disclosed herein, suitable bacteriostatic agents include, but are not limited to, any combination of one or more than two selected from a group consisting of para-hydroxybenzoate compounds (e.g., para-hydroxybenzoate, methyl para-hydroxybenzoate, ethyl para-hydroxybenzoate, propyl para-hydroxybenzoate, butyl para-hydroxybenzoate, heptyl para-hydroxybenzoate, benzyl para-hydroxybenzoate, isobutyl para-hydroxybenzoate, isopropyl para-hydroxybenzoate, or their pharmaceutically acceptable salts), chlorhexidine acetate, metronidazole, silver ions, sorbic acid, benzalkonium chloride, benzethonium chloride, benzyl alcohol, boric acid, ciprofloxacin, cetyltrimethylammonium bromide, hexadecylpyridinium chloride, chlorhexidine, chlorobutanol, chlorocresol, phenol, ethanol, glycerol, hexamidine, imidurea, phenol, phenoxyethanol, benzyl alcohol, mercuric nitrate, polyethylene glycol, and thimerosal. In at least one embodiment of the present disclosure, the bacteriostatic agents include, but are not limited to, any combination of one or more than two selected from a group consisting of para-hydroxybenzoate, methyl para-hydroxybenzoate, ethyl para-hydroxybenzoate, propyl para-hydroxybenzoate, butyl para-hydroxybenzoate, heptyl para-hydroxybenzoate, benzyl para-hydroxybenzoate, isobutyl para-hydroxybenzoate, isopropyl para-hydroxybenzoate, and sodium salts thereof.

In at least one embodiment disclosed herein, the content of the antimicrobial agent in the composition is about 0.01 wt% to about 2 wt%, for example, but not limited to, para-hydroxybenzoate compounds ranging from 0.01 wt% to 2 wt%. In some embodiments, the amount of antimicrobial agent in the composition may be between about 0.05 wt% and about 1 wt%. In some embodiments, the amount of antimicrobial agent in the composition may be between about 0.1 wt% and about 0.5 wt%. In some embodiments, the amount of antimicrobial agent in the composition may be between about 0.1 wt% and about 0.3 wt%. In some embodiments, the amount of antimicrobial agent in the composition may be about 0.01%, about 0.02%, about 0.03%, about 0.04%, about 0.05%, about 0.06%, about 0.07%, about 0.08%, about 0.09%, about 0.1%, about 0.15%, about 0.17%, about 0.2%, about 0.3%, about 0.4%, about 0.5%, about 0.6%, about 0.7%, about 0.8%, about 0.9%, about 1%, about 1.5%, or about 2 wt%. Additionally, the various numerical endpoints mentioned above may be selectively chosen as maximum or minimum values to define the numerical ranges.

In at least one embodiment disclosed herein, suitable gel-forming agents include but not limited to any combination of one or more than two selected from a group consisting of polyacrylic acid, methylcellulose, carboxymethylcellulose, hydroxyethyl cellulose, hydroxyethyl methylcellulose, hydroxypropyl methylcellulose, polyvinyl pyrrolidone, chitosan, carboxymethyl chitosan, hyaluronic acid, alginic acid, aloe vera gel, guar gum, hydroxypropyl guar gum, fenugreek gum, gum arabic, xanthan gum, carrageenan, pectin, gelatin, and gum tragacanth. In at least one embodiment disclosed herein, the gel-forming agent is any combination of one or more than two selected from a group consisting of polyacrylic acid, methylcellulose, carboxymethylcellulose, hydroxyethyl cellulose, hydroxyethyl methylcellulose, hydroxypropyl methylcellulose, and pharmaceutically acceptable salts thereof.

In at least one embodiment disclosed herein, the content of the gel-forming agent in the composition is about 0.3 wt% to about 30 wt%, for example, but not limited to, carboxymethyl cellulose or its sodium salt ranging from about 0.3 wt% to about 30 wt%. In some embodiments, the amount of the gel-forming agent in the composition can range from about 0.5 wt% to about 20 wt%. In some embodiments, the amount of the gel-forming agent in the composition can range from about 1 wt% to about 10 wt%. In some embodiments, the amount of the gel-forming agent in the composition can range from about 2 wt% to about 5 wt%. In some embodiments, the amount of the gel-forming agent in the composition can be about 0.3%, about 0.4%, about 0.5%, about 0.6%, about 0.7%, about 0.8%, about 0.9%, about 1%, about 1.5%, about 2%, about 2.5%, about 2.9%, about 3%, about 4%, about 5%, about 6%, about 7%, about 8%, about 9%, about 10%, about 15%, about 20%, about 25%, or about 30 wt%. Additionally, multiple numerical endpoints mentioned above can be arbitrarily selected as maximum or minimum values to define the numerical range.

In at least one embodiment disclosed herein, adenine, a pharmaceutically acceptable salt thereof, and/or compositions containing it can be administered to a subject via topical or systemic administration.

In at least one embodiment disclosed herein, the effective dose of adenine and/or a pharmaceutically acceptable salt thereof ranges from 0.0001 mg/kg body weight to 50 mg/kg body weight. In at least one embodiment disclosed herein, the effective dose of adenine and/or a pharmaceutically acceptable salt thereof ranges from 0.0006 mg/kg body weight to 50 mg/kg body weight. In at least one embodiment disclosed herein, the effective dose of adenine and/or a pharmaceutically acceptable salt thereof ranges from 0.0006 mg/kg body weight to 0.034 mg/kg body weight and can be used for treating wounds ranging from 1 to 50 square centimeters in size. In at least one embodiment disclosed herein, the effective dose of adenine and/or a pharmaceutically acceptable salt thereof ranges from 0.1 mg/kg body weight to 50 mg/kg body weight. In some embodiments, the effective dose of adenine and/or a pharmaceutically acceptable salt thereof is about 0.0001, about 0.0006, about 0.034, about 0.001, about 0.005, about 0.01, about 0.05, about 0.1, about 0.2, about 0.3, about 0.4, about 0.5, about 0.6, about 0.7, about 0.8, about 0.9, about 1, about 1.5, about 2, about 2.5, about 3, about 3.5, about 4, about 4.5, about 5, about 6, about 7, about 8, about 9, about 10, about 15, about 20, about 25, about 30, about 35, about 40, about 45, or about 50 mg/kg body weight. Additionally, multiple numerical endpoints mentioned above can be arbitrarily selected as maximum or minimum values to define the numerical range.

The following detailed examples are provided in conjunction with the embodiments described above to better illustrate the advantages and effectiveness of the present disclosure and to enable those skilled in the art to practice the disclosure. However, the examples provided should not be construed as limiting the present disclosure.

### Preparation Example

The method for preparing a gel-form composition of adenine at different concentrations is as follows. Firstly, 6.2 g of sodium chloride, 1.6 g of methyl parahydroxybenzoate, 0.2 g of propyl parahydroxybenzoate, and 0.2 g of adenine were dissolved in 1000 mL of distilled water. Then, 30 g of carboxymethyl cellulose was added at a room temperature to obtain a colorless gel with a low concentration of 0.02% adenine. Using the same method, but adjusting the amount of adenine to 20 g, a colorless gel with a concentration of 2% adenine was obtained.

### Example 1

The efficacy of adenine and/or a pharmaceutically acceptable salt thereof as a therapeutic agent for diabetic ulcers was evaluated through a double arm, double-blind study as disclosed in the present disclosure. The groups consist of a 0.02% adenine gel group and a placebo group. The basic information of the subjects is shown in Table 1, while the analysis of wound healing rates based on the Wagner-Meggitt ulcer classification system and initial wound area size is presented in Tables 2 to 4 below, respectively.

**Table 1: Basic information of the subjects**

| | 0.02% concentration adenine gel group | Placebo group | Total |
|---|---|---|---|
| Number of subjects | 90 | 42 | 132 |
| Average age | 61 | 64 | 62 |
| Number of males | 55 | 26 | 81 |
| Number of Females | 35 | 16 | 51 |
| Average body weight (kg) | 70.5 (42.5 to 130.0) | 69.3 (46.0 to 120.0) | 70.0 (42.5 to 130.0) |
| Average BMI | 25.8 (19.1 to 42.5) | 26.2 (18.9 to 42.7) | 26.0 (18.9 to 42.7) |
| Average anklebrachial blood pressure index (ABPI) | 1.09 (0.47 to 1.72) | 1.15 (0.43 to 1.45) | 1.10 (0.43 to 1.72) |

**Table 2: Analysis of Wound Healing Ratio Based on Wagner-Meggitt Ulcer Classification System**

| Wagner-Meggitt Ulcer Classification System | 0.02% concentration adenine gel group | | Placebo group | |
|---|---|---|---|---|
| Grade 1 to Grade 3 | 32/72 | 44.4% | 10/35 | 28.8% |
| Grade 1 and Grade 2 | 24/50 | 48.0% | 5/24 | 20.8% |
| Grade 2 | 23/47 | 48.9% | 5/24 | 20.8% |
| Grade 3 | 8/22 | 38.1% | 5/11 | 45.5% |

**Table 3: Analysis of Wound Healing Ratio Based on Initial Wound Area**

| Initial wound area | 0.02% concentration adenine gel group | | Placebo group | |
|---|---|---|---|---|
| < 1.5 square centimeters | 7/9 | 77.8% | 3/4 | 75.0% |
| 1.5 to 25 square centimeters | 25/60 | 41.7% | 6/29 | 20.7% |
| >25 square centimeters | 0/3 | 0% | 1/2 | 50% |

**Table 4: Analysis of Wound Healing Ratio Based on Wagner-Meggitt Ulcer Classification System and Initial Wound Area**

| | 0.02% concentration adenine gel group | | Placebo group | | P-value |
|---|---|---|---|---|---|
| Healing rate of Grade 1 and Grade 2 ulcers with wound area ranging from 1.5 to 25 square centimeters | 18/41 | 43.9% | 2/20 | 10% | 0.008 |

Based on the results from Table 2 to Table 4, the use of adenine and/or a pharmaceutically acceptable salt thereof as a treatment for diabetic ulcers, specifically Grade 1 and/or Grade 2 ulcers according to the Wagner-Meggitt Ulcer Classification System, or ulcers with a wound area ranging from 1.5 to 25 square centimeters, exhibits remarkably superior effects in accelerating and enhancing wound healing.

### Example 2

Similarly, in a double arm, double-blind study as described in Example 1, the present disclosure evaluated the effects of the disclosed adenine and/or a pharmaceutically acceptable salt thereof as a medicament for treating the diabetic ulcers. The complete wound healing rate from week 0 to week 16 was analyzed and presented in Table 5 below.

**Table 5: Complete wound healing rate from week 0 to week 16**

| Week | adenine gel group | Placebo group |
|---|---|---|
| 0 | 0.00% | 0.00% |
| 1 | 0.00% | 0.00% |
| 2 | 0.00% | 0.00% |
| 4 | 7.32% | 5.00% |
| 6 | 12.20% | 5.00% |
| 8 | 26.83% | 5.00% |
| 10 | 29.27% | 5.00% |
| 12 | 43.90% | 10.00% |
| 14 | 48.78% | 20.00% |
| 16 | 56.10% | 25.00% |

Based on the results from Table 5, it can be concluded that wounds treated with the disclosed adenine and/or a pharmaceutically acceptable salt thereof demonstrated rapid and effective healing starting from the fourth week.

The above examples are provided for illustrative purposes to demonstrate the principles and efficacy of the present disclosure and should not be construed as limiting the scope of the disclosure. Those skilled in the art can modify the above-mentioned examples without departing from the spirit and scope of the present disclosure. Therefore, the scope of the present disclosure should be defined as stated in the claims.

It should be understood that within the scope of the technical content described in this disclosure, various technical features (e.g., those described in specific examples) can be freely combined to form new or preferred technical solutions and applications. For brevity, further elaboration is omitted here. Furthermore, in the numerical ranges formed by the endpoints described in this disclosure, any value falling between the endpoints should be included within the scope of this disclosure, and subranges formed by the endpoints or other values should naturally be included within the scope of this disclosure.

## Claims

1. Use of a pharmaceutical composition comprising adenine and/or a pharmaceutically acceptable salts thereof in the manufacture of a medicament for treating diabetic ulcers.

2. The use of claim 1, wherein the diabetic ulcer is one of Grades 1-3 in Wagner-Meggitt ulcer classification system.

3. The use of claim 2, wherein the diabetic ulcer is Grade 1 or Grade 2 in the Wagner-Meggitt ulcer classification system.

4. The use of claim 2, wherein the diabetic ulcer is located on a skin, a mouth, a gum, a corneal epithelial tissue, or any combination of two or more thereof.

5. The use of claim 2, wherein an area of the diabetic ulcer is in a range from 1.5 to 25 square centimeters.

6. The use of claim 2, wherein the diabetic ulcer is a wound without ischemic and infection, a wound with an ischemic and without infection, a wound without ischemic and with infection, or a wound with ischemic and infection.

7. The use of claim 2, wherein the adenine and/or the pharmaceutically acceptable salts are used for anti-inflammation, increasing collagen secretion, promoting epithelial cell migration, inhibiting scar formation, inhibiting fibroblast growth, or any combination of two or more thereof.

8. The use of claim 2, wherein the adenine and/or a pharmaceutically acceptable salts is a single active ingredient in the pharmaceutical composition.

9. The use of claim 2, wherein the adenine and/or a pharmaceutically acceptable salts in the pharmaceutical composition is in an amount of 0.0001 wt% to 2 wt%.

10. The use of claim 2, wherein an effective dosage of the adenine and/or a pharmaceutically acceptable salts is in a range from 0.0001 mg/kg body weight to 50 mg/kg body weight, and the adenine and/or the pharmaceutically acceptable salts is administered to a subject through a topical administration or a systemic administration.
